# EUROPEAN PATENT APPLICATION

(11) **EP 4 176 870 A1**
(43) Date of publication of application: **10.05.2023**
(21) Application number: 21306558.4
(22) Date of filing: 05.11.2021
(51) Int. Cl.: A61K 9/127, A61K 35/28, A61P 27/02

(54) **EXTRACELLULAR VESICLES OR COMPOSITION THEREOF FOR USE AS A MEDICAMENT, SUCH AS FOR THE TREATMENT OF OCULAR SURFACE DISORDERS**

(71) Applicant: Fondation A de Rothschild, 75019 Paris (FR)
(72) Inventor: GABISON, Eric, 94220 CHARENTON LE PONT (FR)
(74) Representative: Cabinet Beau de Loménie

(57) **Abstract**

The invention provides extracellular vesicles (EVs) or composition thereof for use as a medicament, in particular for use in the treatment or the prevention of an ocular surface blinding disorder (OSBD), wherein said EVs are produced by a genetically modified cell comprising one or more recombinant nucleic acid sequence(s) expressing recombinant Paired Box 6 (rPAX6) and/or recombinant type VII Collagen (rCOL7A1).

## Description

### Technical field

This invention relates to Extracellular vesicles (EVs) produced by a genetically modified cell for use in the treatment or the prevention of an ocular surface blinding disorder (OSBD).

### Background of the invention

The ocular surface is composed of the conjunctival and corneal epithelia. Despite exhibiting different phenotypes, these two tissues are in adjacent continuity. However, while the corneal epithelium is transparent, stratified and non-keratinized, relying on a transparent and avascular stroma, the conjunctival epithelium is semitransparent and relies on an opaque vascularized substantia propria.

The limbus is a specific structure at the periphery of the cornea, creating a transition between the corneal and conjunctival epithelia. The limbus hosts the corneal stem cell niche, which is composed of various cell types, such as mesenchymal stromal cells (MSCs) within a specific extracellular matrix (ECM) and basement membrane that supports Limbal Epithelial Stem Cell (LESC) survival and self-renewal, proliferation and differentiation.

Both corneal cells and ECM properties are important to maintain corneal transparency. Loss of corneal cells or disorganized matrix may lead to a loss of transparency and decrease of vision.

Maintenance of corneal transparency after wounding relies on the capacity of corneal epithelial cells to regenerate from the limbal area and on interactions between epithelial and stromal cells and restoration of the epithelium basement membrane (EBM) in the wounded area.

Rapid epithelial healing is crucial to prevent stromal lysis and/or fibrosis that would hinder visual prognosis. Defective wound healing with chronic EBM disruption leads to abnormal epithelial-stromal interactions, which may lead to a massive induction of matrix proteolytic enzymes responsible for corneal lysis and irregular scarring.

In severe ocular surface blinding disorders (OSBD), corneal epithelial wound healing may be impaired because of inherited or acquired lesions associated with defective EBM healing leading to fibrosis and loss of corneal transparency. Moreover, chronic or recurrent ulcerations result from the progressive destruction of the stem cell niche (limbus), responsible for a limbal stem cell deficiency (LSCD). LSCD can be the consequence of severe trauma (accidental ocular burns, iatrogenic lesions), chronic inflammation (severe dry eye, mucous membrane pemphigoid, toxic epidermal necrolysis) or congenital diseases (congenital aniridia, Recessive dystrophic epidermolysis bullosa). The corneal epithelium progressively undergoes a transdifferentiation toward a conjunctival or cutaneous phenotype, with keratinization and total corneal transparency loss.

Although allogenic corneal graft is successfully used to restore vision by replacing the opaque stroma, it cannot restore the limbal stem cell niche. Therefore, this treatment would fail in patients presenting LSCD. When the LSCD is unilateral, LESC can be transplanted from the fellow eye, prior to the corneal grafting. However, there is no available treatment for patients with bilateral LSCD (including genetic causes of LSCD).

More generally, there is a need of efficient methods for treating ocular surface blinding disorders (OSBD), such as conditions associated with corneal ulceration, conditions associated with conjunctivalization of the cornea, and/or conditions associated with fibrosis of the cornea.

Methods of treatment using autologous epithelial cells from other origin have been developed, such as cultivated oral mucosal epithelial transplantation (COMET). Despite an attractive concept, COMET rarely achieves significant and long-lasting improvement in vision. Moreover, COMET could induce corneal stromal neovascularization that is the major risk factor of corneal graft rejection if subsequently performed [1] [2] [3].

Promising cell therapies using MSCs are under way but present several drawbacks that include the need of repeated injections or surgeries, and the impossibility to control cell survival and migration.

Therapies using extracellular vesicles (EVs) derived from MSCs are also disclosed for treating corneal scarring. EVs derived from MSCs share the bioactive properties of their parental cells and therefore represent flexible, ready to use, off-the-shelf alternatives to stem cell therapy for tissue regeneration, immune modulation, or drug delivery, without the risk of allogenic reaction and with limited side effects.

WO2019/169380 discloses that exosomes derived from corneal MSCs, adipose derived MSCs, umbilical cord MSCs, or bone marrow derived MSCs have the ability to reduce the scar of a mouse model of corneal wounding. One clinical trial using Exosomes from umbilical cord MSCs to treat ocular surface graft versus host disease is underway (NCT04213248).

The present invention provides improved therapies to the use of extracellular vesicles (EVs) for the treatment of OSBD.

### Summary of the invention

The present invention provides an advanced therapy medicinal product for patients with OSBD, combining advantages of EVs therapy and gene therapy using EVs produced by genetically modified cells, especially from mesenchymal stromal cells.

In a first aspect, the invention provides extracellular vesicles (EVs) or composition thereof for use as a medicament, wherein said EVs are produced by a genetically modified cell comprising one or more recombinant nucleic acid sequence(s) expressing recombinant Paired Box 6 (rPAX6) and/or recombinant type VII Collagen (rCOL7A1), and
wherein said EVs contain (i) rPAX6 proteins and/or rPAX6 mRNAs; and/or (ii) rCOL7A1 proteins and/or rCOL7A1 mRNAs.

In a second aspect, the invention provides extracellular vesicles (EVs) or composition thereof for use in the treatment or the prevention of an ocular surface blinding disorder (OSBD),
wherein said EVs are produced by a genetically modified cell comprising one or more recombinant nucleic acid sequence(s) expressing recombinant Paired Box 6 (rPAX6) and/or recombinant type VII Collagen (rCOL7A1); and
wherein said EVs contain (i) rPAX6 proteins and/or rPAX6 mRNAs; and/or (ii) rCOL7A1 proteins and/or rCOL7A1 mRNAs.

### Detailed description of the invention

### Definitions

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

The term "extracellular vesicles" or "EVs" means lipid bound vesicles secreted by cells into the extracellular space. EVs have a diameter from near the size of the smallest physically possible unilamellar liposome (around 20-30 nanometers) to as large as 10 microns or more, although the vast majority of EVs are smaller than 200 nm. EVs are secreted by a number of different cell types for communicating with other cells via the proteins and nucleic acids they carry. Depending on their cellular origin, EVs carry a uniquely distinct profile of proteins, lipids and polynucleotides, which can trigger signaling pathways in other cells and/or transfer EVs products into other cells by EVs fusion with cellular plasma membranes or by endocytosis. EVs can mediate intercellular communication by transferring membrane and cytosolic proteins, lipids, and nucleic acids between cells. These transferred molecules are functional in the recipient cells. The three main subtypes of EVs are microvesicles (MV), exosomes, and apoptotic bodies, which are differentiated based upon their biogenesis, release pathways, size, content, and function.

"PAX6", for "paired box 6", also known as AN, AN1, AN2, FVH1, MGDA, WAGR, ASGD5 or D11S812E, is a protein that in humans is encoded by the *PAX6gene.* PAX6 is a transcription factor acting as a key regulator of eye development. PAX6 deficiency is associated to ocular defects, such as aniridia. Both corneal epithelial and corneal stromal stem cells express high levels of PAX6 in their limbal stem cell niche [4][5].

"COL7A1", for "Collagen Type VII Alpha 1 Chain", also known as EBDCT, EBD1, EBR1 or NDNC8 is a protein that in humans is encoded by the *COL7A1* gene. It functions as an anchoring fibril between the external epithelia and the underlying stroma. Mutations in this gene are associated with all forms of dystrophic epidermolysis bullosa. In the absence of mutations, however, an autoimmune response against type VII collagen can result in an acquired form of this disease called epidermolysis bullosa acquisita.

The term "medium" or "culture medium", as used herein, refers to any substance or preparation used for the cultivation of living cells, including the components of the environment surrounding the cells. The medium can be any medium adequate for culturing cells. In a particular embodiment, the medium does not comprise any type of sera, including fetal bovine serum, bovine serum (BS), calf serum (CS), fetal calf serum (PCS), newborn calf serum (NCS), goat serum (GS), horse serum (HS), porcine serum, sheep serum, rabbit serum, rat serum (RS), platelet lysate, such as human platelet lysate.

The term "pharmaceutically acceptable" means approved by a regulatory agency of the Federal or a state government or listed in the U.S. or European Pharmacopeia or other generally recognized pharmacopeia for use in animals and Humans.

The terms "pharmaceutically acceptable excipient", or "pharmaceutically acceptable carrier, or "pharmaceutically acceptable diluent", or "pharmaceutically acceptable vehicle" used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation.

The term "pharmaceutical composition", as used herein, means a composition comprising pharmaceutically acceptable carrier. For example, a carrier can be a diluent, adjuvant, excipient, or vehicle with which the therapeutic is administered. In the context of the invention, "pharmaceutical composition" refers to a composition comprising a therapeutically effective amount of EVs and at least one pharmaceutically acceptable carrier. In some embodiments, the pharmaceutical composition comprises EVs, such as isolated EVs, in combination with a non-naturally occurring pharmaceutically acceptable carrier.

The term "Recombinant" is used consistently with its usage in the art to refer to or denoting an organism, cell, protein or genetic material formed or produced by recombination. For example, a recombinant nucleotide sequence (or transgene) is created by a process that involves the human intervention and/or is generated from a nucleic acid that was created by human intervention (e.g., by one or more cycles of replication, amplification, transcription, etc.). For example, a recombinant protein is one that is coded by a recombinant nucleotide sequence. For example, a recombinant cell is a genetically modified cell comprising one or more recombinant nucleic acid sequence(s) (or transgene) expressing one or more recombinant protein(s).

The term "subject", "patient" or "individual", as used herein, refers to a human or non-human mammal (such as a rodent (mouse, rat), a feline, a canine, or a primate). Preferably, the subject is a human, man or woman.

### First and second medical use

In a first aspect, the invention provides extracellular vesicles (EVs) or composition thereof for use as a medicament,
wherein said EVs are produced by a genetically modified cell comprising one or more recombinant nucleic acid sequence(s) expressing recombinant Paired Box 6 (rPAX6) and/or recombinant type VII Collagen (rCOL7A1), and
wherein said EVs contain (i) rPAX6 proteins and/or rPAX6 mRNAs; and/or (ii) rCOL7A1 proteins and/or rCOL7A1 mRNAs.

In a second aspect, the invention provides extracellular vesicles (EVs) or composition thereof for use in the treatment or the prevention of an ocular surface blinding disorder (OSBD),
wherein said EVs are produced by a genetically modified cell comprising one or more recombinant nucleic acid sequence(s) expressing recombinant Paired Box 6 (rPAX6) and/or recombinant type VII Collagen (rCOL7A1); and
wherein said EVs contain (i) rPAX6 proteins and/or rPAX6 mRNAs; and/or (ii) rCOL7A1 proteins and/or rCOL7A1 mRNAs.

In some embodiments, EVs are produced by a genetically modified cell comprising a recombinant nucleic acid sequence expressing rPAX6 isoform A. The amino acid sequence of rPAX6 isoform A is SEQ ID NO: 1. According to these embodiments, the genetically modified cell comprises one or more recombinant nucleic acid sequence(s) expressing rPAX6 isoform A, such as one or more recombinant nucleic acid sequence(s) of SEQ ID NO: 2.

In some embodiments, EVs are produced by a genetically modified cell comprising a recombinant nucleic acid sequence expressing rPAX6 isoform B. The amino acid sequence of rPAX6 isoform B is SEQ ID NO: 3. According to these embodiments, the genetically modified cell comprises one or more recombinant nucleic acid sequence(s) expressing rPAX6 isoform B, such as one or more recombinant nucleic acid sequence(s) of SEQ ID NO: 4.

In some embodiments, EVs are produced by a genetically modified cell comprising a recombinant nucleic acid sequence expressing rCOL7A1 having the amino acid sequence SEQ ID NO: 5. According to these embodiments, the genetically modified cell comprises one or more recombinant nucleic acid sequence(s) expressing rCOL7A1 of SEQ ID NO: 5, such as one or more recombinant nucleic acid sequence(s) of SEQ ID NO: 6.

In some embodiments, the OSBD is a condition associated with corneal ulceration, a condition associated with conjunctivalization of the cornea and/or a condition associated with fibrosis of the cornea.

In some embodiments, the condition is limbal stem cell deficiency (LSCD).

In some embodiments, the LSCD is the consequence of:
- a lesion of the cornea, such as burn of the cornea (e.g. chemical burn), physical lesions of the cornea (e.g. corneal limbal scarring);
- a chronic inflammation of the cornea, such as severe dry eye (e.g. due to Sjögren's Syndrome), mucous membrane pemphigoid, toxic epidermal necrolysis; or
- a congenital diseases of the cornea, such as congenital aniridia, aniridia-associated keratopathy (AAK) or recessive dystrophic epidermolysis bullosa (RDEB).

In some other embodiments, the condition is neurotrophic keratitis, recurrent corneal erosion syndrome or mild dystrophic epidermolysis bullosa.

In some other embodiments, the condition is an ocular GVHD (Graft Versus Host Disease).

In some embodiments, the EVs or composition thereof as disclosed herein have the property to restore the structure of the corneal epithelial cells, to restore the interactions between epithelial and stromal cells and/or to restore the epithelium basement membrane. The EVs or composition thereof as disclosed herein may also promote rapid epithelial healing, favor EBM maturation and/or strengthens epithelial adhesion to prevent recurrences.

In some embodiments, the EVs or composition thereof as disclosed herein also prevent opacification of the cornea and/or improve corneal healing.

In some embodiments, the EVs or composition thereof as disclosed herein also promote corneal tissue regeneration and/or corneal tissue healing.

EVs or composition thereof as disclosed herein may also have one or more of the following properties, but not limited to:
- Slowing and/or preventing degeneration of epithelial stem cells,
- reduction of the scar of corneal wounding,
- Corneal wound repair,
- Treatment and prevention of corneal scarring,
- Preservation of cornea transparency,
- Prevention and treatment of cornea ulceration and erosion,
- Prevention and delay of LSCD,
- Improvement of epithelium attachment to basement membrane.
- Limition of associated pain,
- Prevention of conjunctive fibrosis, including symblepharon and fornix foreshortening, and/or
- Prevention of ocular surface conjunctivalization and keratinization.

This represents a groundbreaking therapeutic innovation for patients with OSBD.

In some embodiments, the EVs are produced by a genetically modified cell, such as a genetically modified mesenchymal stromal cell, comprising one or more recombinant nucleic acid sequence(s) expressing recombinant Paired Box 6 (rPAX6), and said EVs contain rPAX6 proteins and/or rPAX6 mRNAs. In these embodiments, the EVs or composition thereof for use according to the invention are particularly useful in the treatment of corneal damages due to Sjögren's syndrome, congenital aniridia, aniridia-associated keratopathy (AAK), Ocular GVHD, Stevens Johnson Lyell syndrome and all other disorders associated with severe dry eyes.

In some embodiments, the EVs are produced by a genetically modified cell, such as a genetically modified mesenchymal stromal cell, comprising one or more recombinant nucleic acid sequence(s) expressing recombinant type VII Collagen (rCOL7A1), and said EVs contain rCOL7A1 proteins and/or rCOL7A1 mRNAs. In these embodiments, the EVs or composition thereof for use according to the invention are particularly useful in the treatment of RDEB, recurrent corneal erosions syndromes, neurotrophic keratitis.

In some embodiments, the EVs are produced by a genetically modified cell, such as a genetically modified mesenchymal stromal cell, comprising one or more recombinant nucleic acid sequence(s) expressing recombinant Paired Box 6 (rPAX6) and recombinant type VII Collagen (rCOL7A1), and said EVs contain (i) rPAX6 proteins and/or rPAX6 mRNAs; and (ii) rCOL7A1 proteins and/or rCOL7A1 mRNAs. In this embodiment, the EVs or composition thereof for use according to the invention are particularly useful in the treatment of to Sjögren's syndrome, congenital aniridia, aniridia-associated keratopathy (AAK), Ocular GVHD, Stevens Johnson Lyell syndrome, RDEB, recurrent corneal erosions syndroms, neurotrophic keratitis, and all other disorders associated with sever dry eyes.

The EVs or composition thereof as disclosed herein are delivery vehicles (or cargos) to deliver Col7A1 and/or PAX6 proteins/mRNA, into cells, tissue, or organs, preferably into the corneal tissue cells. The EVs or composition thereof as disclosed herein are internalized into cells of the tissue or organs, thereby delivering (i) rPAX6 proteins and/or rPAX6 mRNAs; and/or (ii) rCOL7A1 proteins and/or rCOL7A1 mRNAs into cells of the tissue or organs, preferably into the corneal tissue cells. Therefore, in some embodiments, the EVs are used to treat cells, tissue, or organs damages, such as corneal damages, in a patient by administering to said patient a therapeutically effective amount of said EVs or composition thereof.

In some embodiments, EVs contain a therapeutically effective amount of (i) rPAX6 proteins and/or rPAX6 mRNAs; and/or (ii) rCOL7A1 proteins and/or rCOL7A1 mRNAs.

According to the invention, the amount of (i) PAX6 proteins and/or PAX6 mRNAs (including recombinant and naturally produced by the cell); and/or (ii) COL7A1 proteins and/or COL7A1 mRNAs (including recombinant and naturally produced by the cell) in the EVs produced by the genetically modified cell as disclosed in the present invention is higher compared to the amount of (i) PAX6 proteins and/or PAX6 mRNAs (i.e. naturally produced by the cell); and/or (ii) COL7A1 proteins and/or COL7A1 mRNAs (i.e. naturally produced by the cell) in the EVs produced by a non-genetically modified cell.

In some embodiments, the genetically modified cell is an induced pluripotent stem cell (iPS).

In some embodiments, the genetically modified cell is a mesenchymal stromal cell (MSC), such as a bone marrow-derived MSC (BMMSC), a dental pulp MSC, an adipose tissue MSC, a cornea limbal MSC, a placental MSC, or an umbilical cord MSC (UCMSC), preferably a UCMSC. UCMSC are particularly advantageous due to the innate pro-healing, anti-fibrotic and anti-inflammatory properties of EVs derived from a UCMSC.

The EVs are produced by a genetically modified cell, such as a mesenchymal stromal cell (e.g. a MSC). Where the cell, such as a MSC, is in cell culture, the EVs may be secreted into the cell culture medium. The EVs may be formed by inward budding of the endosomal membrane. The EVs may comprise one or more polynucleotides or proteins present in the cell that produces the EVs, such as a protein characteristic or specific to the MSC. EVs as disclosed herein contain (i) rPAX6 proteins and/or rPAX6 mRNAs; and/or (ii) rCOL7A1 proteins and/or rCOL7A1 mRNAs.

In some embodiments, the protein and/or mRNA loading of rPAX6 and/or rCOL7A1 into EVs is increased by modulating shear forces, such as implementing "Turbulence technologies" [6]. Other methods can also force the packaging of rPAX6 and/or rCOL7A1 protein/mRNA into EVs, such as electroporation of exogenous rPAX6 and/or rCOL7A1 protein/mRNA [7] or sonication of exogenous rPAX6 and/or rCOL7A1 protein/mRNA. Cellular-nanoporation was also described to encapsulate selected mRNA in EVs [8].

The recombinant cell that produces the EVs is modified to contain the so-called one or more recombinant nucleic acid sequence(s) expressing rPAX6 and/or Col7A1 rCOL7A1 placed under control of proper regulatory sequences for its expression, such as a promoter, an enhancer, etc. Natural or synthetic 5'-UTR and/or 3'-UTR can be added to the recombinant cDNA of COL7A1 and/or PAX6 to potentiate an enrichment of mRNA of rCOL7A1 and/or rPAX6 loading in EV.

Methods for introducing a recombinant nucleotide sequence into cells are known in the art and including, as non-limiting examples, stable transduction methods wherein the recombinant nucleotide sequence is integrated into the genome of the cell (e.g. recombinant viral vector-mediated methods, liposomes, microinjection, electroporation, particle bombardment, polyethyleneimine). Transduction methods, such as electroporation and polyethyleneimine, are preferably performed with a sleeping beauty transposon system. The recombinant nucleotide sequence may be included in a vector, more particularly a plasmid or a viral vector, in view of being expressed into cells. In a preferred embodiment, the method for introducing a recombinant nucleotide sequence encoding rPAX6 and/or rCOL7A1 into cells is a recombinant viral vector-mediated methods (e.g. lentivirus), electroporation or polyethyleneimine.

The cell that produces EVs may be immortalized. Therefore, in some embodiments, the EVs are produced by an immortalized cell line. Cells can be immortalized using conventional techniques, such as using a lentiviral construction overexpressing the oncogene cMYC, SV40 antigen or the telomerase reverse transcriptase (TERT). The immortalization of cells offers the possibility- for constant supply of EVs having defined properties, less variability in the EVs production, elimination of variation of donors and a safe technology for treating subjects. The immortalization of the cells offers the possibility for constant supply of EVs, less variability in the EVs production, elimination of variation of donors and a safe technology for treating patients.

The EVs may be isolated, for example from a culture medium of cells producing the EVs, based on any property of the EVs. For example, the EVs may be isolated based on molecular weight, size, shape, composition or biological activity. Any appropriate techniques can be used to isolate the EVs, such as centrifugation, filtration, chromatography, size exclusion and/or affinity, preferably tangential flow filtration.

In some embodiments, the EVs or composition thereof is administered in the eye and/or by subconjunctival injection.

Composition comprising the EVs can be formulated as a pharmaceutical composition in a variety of forms adapted to the chosen route of administration, for example, in an ocular infusion, such as eye drops.

The pharmaceutical composition may be administered in a variety of ways, for example intraocular administration. The composition can be prepared in water, optionally mixed with a nontoxic surfactant. The pharmaceutical composition can also be prepared in glycerol, liquid polyethylene glycols, triacetin, and mixtures thereof and in oils. The pharmaceutical composition can also be prepared in biologic mixtures, such as Warthon Jelly. Under ordinary conditions of storage and use, the pharmaceutical composition can contain a preservative to prevent the growth of microorganisms.

The pharmaceutical composition suitable for injection or infusion can include sterile aqueous solutions or dispersions or sterile powders comprising the EVs which are adapted for the extemporaneous preparation of sterile infusible composition. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof.

Useful liquid carriers include water, alcohols or glycols or water/alcohol/glycol blends, in which the EVs ca be dissolved or dispersed at effective levels, optionally with the aid of non-toxic surfactants. Adjuvants such as additional antimicrobial agents can be added to optimize the properties for a given use.

Effective dosages and routes of administration of EVs or composition thereof of the invention are conventional. The exact amount (effective dose) of the EVs will vary from subject to subject, depending on, for example, the species, age, weight and general or clinical condition of the subject, the severity or mechanism of any disorder being treated, the particular agent or vehicle used, the method and scheduling of administration, and the like. A therapeutically effective dose can be determined empirically, by conventional procedures known to those of skill in the art. For example, an, effective dose can be estimated initially either in cell culture assays or in suitable animal models. The animal model may also be used to determine the appropriate concentration ranges and routes of administration. Such information can then be used to determine useful doses and routes for administration in humans. A therapeutic dose can also be selected by analogy to dosages for comparable therapeutic agents.

The particular mode of administration and the dosage regimen will be selected by the attending clinician, taking into account the particulars of the case (e.g. the subject, the disease, the disease state involved, and whether the treatment is prophylactic). Treatment may involve daily or multi-daily doses EVs or composition thereof over a period of a few days to months.

In a particular preferred embodiment, the pharmaceutical composition of the invention is formulated as an ophthalmic formulation for administration to the eye and for this reason have formulation elements specifically selected for this purpose. For example, the most widely used ophthalmic buffer solutions are boric acid vehicle and Sorensen's modified phosphate buffer. The boric acid vehicle is a 1.9% solution of boric acid in purified water or preferably sterile water. It is isotonic with tears. It has a pH of approximately 5 and is useful when extemporaneously compounding ophthalmic solutions of drugs that are most stable at acid pH. Optionally, the buffer solution is convenient to use as a tonicity adjustor. In circumstances when an ophthalmic solution without a buffer is desired, any compatible salt or non-electrolyte that is approved for ophthalmic products may be used. Sodium chloride, sodium nitrate, sodium sulfate, and dextrose are common neutral tonicity adjustors.

The use of preservatives is common ophthalmic solutions in order to prevent the growth of, or to destroy, microorganisms accidentally introduced when the container is opened during use. Benzyl alcohol, thimerosal and the parabens are the preservatives commonly found in ophthalmic solutions.

In addition, certain active ingredient(s) may be susceptible to oxidative degradation. If oxidation is a problem, an antioxidant can be included. Sodium metabisulfite and Sodium bisulfite are acceptable for this purpose in drug preparations.

Finally, an increase in the viscosity of ophthalmic products will result in a longer residence time in the eye, providing a longer time for drug absorption and effect. Numerous viscosity enhancing materials can be used, among winch methylcellulose is a common example.

In some embodiments, the EVs or composition thereof as disclosed herein are delivered by direct injection into the corneal stroma. In other embodiments, the EVs or a composition thereof are delivered by coating an EVs containing hydrogel in the inner surface of a contact lens and contacting an eye with the contact lens. Such embodiments can employ a number of reagents and/or method steps that are performed in treatment of OSBD.

### Description of the figures

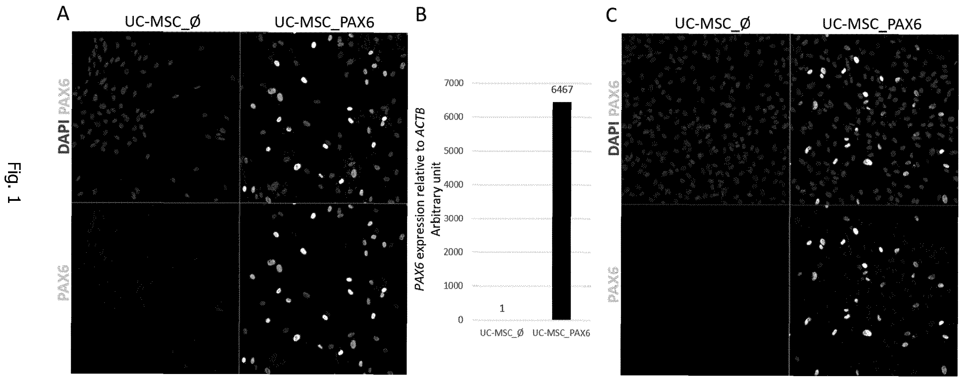

Figure 1: rPAX6 expression in umbilical cord mesenchymal stromal cells (UC-MSC). A) UC-MSC are transduced (UC-MSC_PAX6) or not (UC-MSC_Ø) by a lentiviral vector allowing stable PAX6 expression. 3 days post transduction UC-MSC were immunostained for PAX6 (orange) and nucleic acids are stained using DAPI (blue), ∼65% of UC-MSC were stained for PAX6. B) *PAX6* mRNA relative expression quantified by RT-qPCR between UC-MSC_0 and UC-MSC_PAX6, 15 days post lentiviral transduction. C) UC-MSC were electroporated (UC-MSC_PAX6) or not (UC-MSC_Ø) by a pFAR4 plasmid allowing stable PAX6 expression thanks to sleeping beauty transposon system. 7 days post transduction UC-MSC were immunostained for PAX6 (orange) and nucleic acids are stained using DAPI (blue), ∼15% of UC-MSC were stained for PAX6.

### Examples

### Example 1: Material and methods

### 1.1 UC-MSC culture

MSCs were isolated from the UC Wharton's jelly of a single healthy donor, using the explant method (PMID: 28844128). Cells were grown in an incubator at 37°C with 5% of CO₂ in aMEM (Minimum Essential Medium) containing, 10% of serum, antibiotic & antimycotic and 1ng/ml of bFGF (Fibroblast Growth Factor).

### 1.2 Plasmids design and electroporation

PAX6 isoform A and PAX6 isoform B were independently introduced into a pFAR4 vector [9]. pFAR4 vector, a miniplasmid devoid of antibiotic resistance marker, was used as a gene vector to deliver the *Sleeping Beauty (SB)* transposon system. pFAR4 vector successfully demonstrated its safety and efficiency for gene delivery and is currently used in the clinic (ACTRN12618001556235). SB system is composed of a two inverted terminal repeats sequences (ITRs) surrounding the gene of interest (GOI) and its promoter (CAG). ITRs were excised from the transposon plasmid and randomly inserted into the genome by the *SB* transposase encoded by a second pFAR4 plasmid, allowing stable expression in cells.

After trypsinization, 1.10⁶ UC-MSC were resuspended in 500µL of aMEM without serum and antibiotic. 20µg of pFAR4 plasmids was added to the cells suspension and incubated 5min on ice. The mixture was transferred in electroporation cuvette (4mm wide). The cuvette was then inserted in a BTX ECM830 electroporation system. 1 pulse of 50 ms at 200 volt was applied. Cells in the cuvette were then incubated 10 min in an incubator at 37°C, 5% of CO₂ before to be seeded in cell culture flask and cells were grown as routinely. Using this procedure 15% of transfection rate was obtained (Figure 1C).

### 1.3 Lentiviral vector design and transduction

A nucleic acid coding *PAX6 isoform A* and a nucleic acid coding *PAX6 isoform B* were independently introduced in a lentiviral vector under the control of EF1a promoter. The EF1a promoter ensures ubiquitous and stable expression of the cassette. UC-MSCs were resuspended in αMEM with 10% of serum and 10µg/ml of Polybrene. Lentiviral particle were mixed with cells at MOI 50. After 48h the medium containing viral particles was washed and replaced by medium used for routine UC-MSC culture. Using this procedure 65% of transduction was obtained.

A nucleic acid coding COL7A1 was introduced in a lentiviral vector under the control of EFS promoter. The EFS promoter ensures ubiquitous and stable expression of the cassette. UC-MSCs were transduced and a rate of 60% of positive cells was obtained (Figure 1A).

### 1.4 EVs production

UC-MSCs stably expressing the GOI were grown as routinely until cells reach 80%-90% of confluency. Cells were then rinsed 3x with the starvation medium (aMEM without serum and dFGF). Cells were incubated for 2h at 37°C, 5% CO₂ in starvation medium, the medium was replaced on more time and cells were incubated for 48h. Additionally, in order to increase EV production by cells, shear forces is increase using EVerZom "Turbulence technologies" [6].

The conditioned medium was harvested and centrifuged 5min at 500g. Evaluation of EV content and size distribution in conditioned medium was assessed by Nanoparticle Tracking Analysis (NTA) and conditioned medium was stored at -80°C.

Additionally, after harvesting the conditioned medium, a sample of cells was subjected to cytometry analysis to get the rate of cells expressing the GOI, and, a RNA sample was prepared and analyzed by RT-qPCR in order to get the expression of the GOI in the whole cell population (Figure 1B).

### 1.5 EVs isolation

EVs were isolated by tangential flow ultrafiltration (TFF). First, conditioned medium was cleared (0.45 filtration) and subjected to TFF. We used a KFR2i device (Spectrum Laboratories) and a hollow fiber filtration system allowing ultrafiltration of sample volumes from 50mL up to 10L. Control of continuous feed rate allows the filtration to operate at constant shear rate. Filtration was controlled by pressure sensors ensuring maximum reproducibility of the process.

Starting from a 1.1L solution of conditioned medium, it took about 4h to isolate EVs with an expected 90% yield. The EV were isolated in starvation medium, which was then exchange with another liquid medium such as defined saline solution.

### 1.6 Analysis of EV content

Each batch of EVs is controlled for the content of the mRNA and protein of interest by RT-qPCR and ELISA respectively.

### 1.7 In vitro analysis of PAX6 transcriptional activity, in target cells, incubated with EVs produced by UC-MSC expressing rPAX6 according to the invention

In order to assess PAX6 transcriptional activity a fluorescence reporter was built. A repetition of consensus sequences of PAX6 binding sites are inserted front of a CMV minimal promoter followed by GFP and luciferase. The activation of the promoter requires PAX6 binding. The GFP fluorescence is quantified by flow cytometry, microscopy or plate reader and the luminescence by plate reader, allowing quantitative analysis of PAX6 transcriptional activity in target cells [10]. The reporter was introduced in corneal cell lines (HTK cells: Stromal corneal cell line and hTCEpi: Epithelial corneal cell line) and also in non-specific cell lines (such as HeLa cells).

### 1.8 In vitro functional rescue of PAX6 haploinsufficiency in corneal cells

PAX6 is knocked down on a single allele in an immortalized corneal epithelial cell line by Crispr/cas9 [11]. EVs produced by UC-MSC expressing rPAX6 according to the invention are incubated with these cells and PAX6, KRT3, KRT12 and MMP9 expression is assessed by RT-qPCR and immunofluorescence [11] [12] [13] in order to determine the transcriptional rescue in haploinsufficient cells. Additionally, using the Incucyte^{®} Scratch Wound Assays, the functionality of the cells is assessed, based on the rescue of cell migration [11].

### 1.9 In vitro functional rescue of myofibroblast transformation (inhibition of fibrosis)

An immortalized corneal stromal cell line is treated with TGFβ inducing a myofibroblast transformation [14] which is characterized by the expression of ACTA2. The functional inhibition of said myofibroblast transformation by EVs produced by UC-MSC expressing rPAX6 according to the invention, is assessed by the decrease of ACTA2 expression by immunofluorescence and RT-qPCR [15].

### 1.10 In vivo functional rescue of Col7a1 deficiency in mice.

In a Knock-in (KI) mouse model of Col7a1 deficiency having reduced or missing Col7a1 expression at the basement membrane (skin, oesophagus and cornea). The mouse model is supplied with EVs produced by UC-MSC expressing Col7a1 according to the invention and the rescue of Col7a1 localisation is assessed by immunostaining of the cornea.

### References cited under the form "[reference number]"

[1] Satake et al. Ophthalmology, 118,1524-1530 (2011)
[2] Sugiyama et al. The American Society of Gene & Cell Therapy, vol. 22 no. 8 (2014)
[3] Hayashida et al. Investigative Ophthalmology & Visual Science, vol. 46, no. 5 (2005)
[4] Funderburgh et al. The FASEB Journal (2005)
[5] Du et al. Stem Cells, Vol. 23, 1266-1275 (2005)
[6] WO/2019/002608
[7] Alvarez-Erviti, L. et al. Nat. Biotechnol. 29, 341-345 (2011)
[8] Yang, Z. et al. Nat. Biomed. Eng. 4, 69-83 (2020)
[9] Pastor et al. Molecular Therapy: Nucleic Acids Vol. 11 June (2018)
[10] Oved et al. Biochemical and Biophysical Research Communications, Vol. 582, Pages 100-104 (2021)
[11] Roux et al. Stem Cells, Vol. 36:1421-1429 (2018)
[12] Latta et al. Exp Eye Res, Vol. 167, 100-109 (2018)
[13] Sivak et al. Developmental Biology, Vol. 222, Issue 1, 41-54 (2000)
[14] Jesper et al. Investigative Ophthalmology & Visual Science, Vol. 44, 1850-1858 (2003)
[15] Shojaati et al. Stem Cells Transl Med, Vol. 8, 1192-1201 (2019)

### Sequence listing

| SEQ ID | Reference | Sequence |
|---|---|---|
| 1 | Amino acid sequence of rPAX6 isoform A | |
| 2 | Nucleic acid coding rPAX6 isoform A | atgcagaacagtcacagcggagtgaatcagctcggtggtgtctttgtcaacgggcggcca ctgccggactccacccggcagaagattgtagagctagctcacagcggggcccggccgtg cgacatttcccgaattctgcaggtgtccaacggatgtgtgagtaaaattctgggcaggtatt acgagactggctccatcagacccagggcaatcggtggtagtaaaccgagagtagcgact ccagaagttgtaagcaaaatagcccagtataagcgggagtgcccgtccatctttgcttggg aaatccgagacagattactgtccgagggggtctgtaccaacgataacataccaagcgtgt catcaataaacagagttcttcgcaacctggctagcgaaaagcaacagatgggcgcagac ggcatgtatgataaactaaggatgttgaacgggcagaccggaagctggggcacccgccc tggttggtatccggggacttcggtgccagggcaacctacgcaagatggctgccagcaaca ggaaggagggggagagaataccaactccatcagttccaacggagaagattcagatgag gctcaaatgcgacttcagctgaagcggaagctgcaaagaaatagaacatcctttacccaa gagcaaattgaggccctggagaaagagtttgagagaacccattatccagatgtgtttgccc gagaaagactagcagccaaaatagatctacctgaagcaagaatacaggtatggttttcta atcgaagggccaaatggagaagagaagaaaaactgaggaatcagagaagacaggcc agcaacacacctagtcatattcctatcagcagtagtttcagcaccagtgtctaccaaccaatt ccacaacccaccacaccggtttcctccttcacatctggctccatgttgggccgaacagacac agccctcacaaacacctacagcgctctgccgcctatgcccagcttcaccatggcaaataac ctgcctatgcaacccccagtccccagccagacctcctcatactcctgcatgctgcccaccag cccttcggtgaatgggcggagttatgatacctacacccccccacatatgcagacacacatg aacagtcagccaatgggcacctcgggcaccacttcaacaggactcatttcccctggtgtgt cagttccagttcaagttcccggaagtgaacctgatatgtctcaatactggccaagattacag taa |
| 3 | Amino acid sequence of rPAX6 isoform B | MQNSHSGVNQLGGVFVNGRPLPDSTRQKIVELAHSGARPCDISRILQT HADAKVQVLDNQNVSNGCVSKILGRYYETGSIRPRAIGGSKPRVATPEV VSKIAQYKRECPSIFAWEIRDRLLSEGVCTNDNIPSVSSINRVLRNLASE KQQMGADGMYDKLRMLNGQTGSWGTRPGWYPGTSVPGQPTQDGC QQQEGGGENTNSISSNGEDSDEAQMRLQLKRKLQRNRTSFTQEQIEA LEKEFERTHYPDVFARERLAAKIDLPEARIQVWFSNRRAKWRREEKLRN QRRQASNTPSHIPISSSFSTSVYQPIPQPTTPVSSFTSGSMLGRTDTALT NTYSALPPMPSFTMANNLPMQPPVPSQTSSYSCMLPTSPSVNGRSYDT YTPPHMQTHMNSQPMGTSGTTSTGLISPGVSVPVQVPGSEPDMSQY WPRLQ |
| 4 | Nucleic acid codinq | atgcagaacagtcacagcggagtgaatcagctcggtggtgtctttgtcaacgggcggcca ctgccggactccacccggcagaagattgtagagctagctcacagcggggcccggccgtg |
| | rPAX6 isoform B | cgacatttcccgaattctgcagacccatgcagatgcaaaagtccaagtgctggacaatcaa aacgtgtccaacggatgtgtgagtaaaattctgggcaggtattacgagactggctccatc agacccagggcaatcggtggtagtaaaccgagagtagcgactccagaagttgtaagcaa aatagcccagtataagcgggagtgcccgtccatctttgcttgggaaatccgagacagatta ctgtccgagggggtctgtaccaacgataacataccaagcgtgtcatcaataaacagagttc ttcgcaacctggctagcgaaaagcaacagatgggcgcagacggcatgtatgataaacta aggatgttgaacgggcagaccggaagctggggcacccgccctggttggtatccggggac ttcggtgccagggcaacctacgcaagatggctgccagcaacaggaaggagggggaga gaataccaactccatcagttccaacggagaagattcagatgaggctcaaatgcgacttca gctgaagcggaagctgcaaagaaatagaacatcctttacccaagagcaaattgaggccc tggagaaagagtttgagagaacccattatccagatgtgtttgcccgagaaagactagcag ccaaaatagatctacctgaagcaagaatacaggtatggttttctaatcgaagggccaaatg gagaagagaagaaaaactgaggaatcagagaagacaggccagcaacacacctagtca tattcctatcagcagtagtttcagcaccagtgtctaccaaccaattccacaacccaccacacc ggtttcctccttcacatctggctccatgttgggccgaacagacacagccctcacaaacacct acagcgctctgccgcctatgcccagcttcaccatggcaaataacctgcctatgcaaccccca gtccccagccagacctcctcatactcctgcatgctgcccaccagcccttcggtgaatgggcg gagttatgatacctacacccccccacatatgcagacacacatgaacagtcagccaatggg cacctcgggcaccacttcaacaggactcatttcccctggtgtgtcagttccagttcaagttcc cggaagtgaacctgatatgtctcaatactggccaagattacagtaa |
| 5 | Amino acid sequence of rCOL7A1 | MTLRLLVAALCAGILAEAPRVRAQHRERVTCTRLYAADIVFLLDGSSSIG RSNFREVRSFLEGLVLPFSGAASAQGVRFATVQYSDDPRTEFGLDALGS GGDVIRAIRELSYKGGNTRTGAAILHVADHVFLPQLARPGVPKVCILITD GKSQDLVDTAAQRLKGQGVKLFAVGIKNADPEELKRVASQPTSDFFFFV NDFSILRTLLPLVSRRVCTTAGGVPVTRPPDDSTSAPRDLVLSEPSSQSL RVQWTAASGPVTGYKVQYTPLTGLGQPLPSERQEVNVPAGETSVRLRG LRPLTEYQVTVIALYANSIGEAVSGTARTTALEGPELTIQNTTAHSLLVA WRSVPGATGYRVTWRVLSGGPTQQQELGPGQGSVLLRDLEPGTDYEV TVSTLFGRSVGPATSLMARTDASVEQTLRPVILGPTSILLSWNLVPEAR GYRLEWRRETGLEPPQKVVLPSDVTRYQLDGLQPGTEYRLTLYTLLEG HEVATPATVVPTGPELPVSPVTDLQATELPGQRVRVSWSPVPGATQYR IIVRSTQGVERTLVLPGSQTAFDLDDVQAGLSYTVRVSARVGPREGSAS VLTVRREPETPLAVPGLRVVVSDATRVRVAWGPVPGASGFRISWSTGS GPESSQTLPPDSTATDITGLQPGTTYQVAVSVLRGREEGPAAVIVARTD PLGPVRTVHVTQASSSSVTITWTRVPGATGYRVSWHSAHGPEKSQLVS GEATVAELDGLEPDTEYTVHVRAHVAGVDGPPASVVVRTAPEPVGRVS RLQILNASSDVLRITWVGVTGATAYRLAWGRSEGGPMRHQILPGNTDS AEIRGLEGGVSYSVRVTALVGDREGTPVSIVVTTPPEAPPALGTLHVVQ RGEHSLRLRWEPVPRAQGFLLHWQPEGGQEQSRVLGPELSSYHLDGL EPATQYRVRLSVLGPAGEGPSAEVTARTESPRVPSIELRVVDTSIDSVTL AWTPVSRASSYILSWRPLRGPGQEVPGSPQTLPGISSSQRVTGLEPGVS YIFSLTPVLDGVRGPEASVTQTPVCPRGLADVVFLPHATQDNAHRAEAT RRVLERLVLALGPLGPQAVQVGLLSYSHRPSPLFPLNGSHDLGIILQRIR DMPYMDPSGNNLGTAVVTAHRYMLAPDAPGRRQHVPGVMVLLVDEPL RGDIFSPIREAQASGLNVVMLGMAGADPEQLRRLAPGMDSVQTFFAVD DGPSLDQAVSGLATALCQASFTTQPRPEPCPVYCPKGQKGEPGEMGLR GQVGPPGDPGLPGRTGAPGPQGPPGSATAKGERGFPGADGRPGSPGR |
| | | AGNPGTPGAPGLKGSPGLPGPRGDPGERGPRGPKGEPGAPGQVIGGE GPGLPGRKGDPGPSGPPGPRGPLGDPGPRGPPGLPGTAMKGDKGDRG ERGPPGPGEGGIAPGEPGLPGLPGSPGPQGPVGPPGKKGEKGDSEDGA PGLPGQPGSPGEQGPRGPPGAIGPKGDRGFPGPLGEAGEKGERGPPGP AGSRGLPGVAGRPGAKGPEGPPGPTGRQGEKGEPGRPGDPAVVGPAV AGPKGEKGDVGPAGPRGATGVQGERGPPGLVLPGDPGPKGDPGDRGP IGLTGRAGPPGDSGPPGEKGDPGRPGPPGPVGPRGRDGEVGEKGDEG PPGDPGLPGKAGERGLRGAPGVRGPVGEKGDQGDPGEDGRNGSPGSS GPKGDRGEPGPPGPPGRLVDTGPGAREKGEPGDRGQEGPRGPKGDPG LPGAPGERGIEGFRGPPGPQGDPGVRGPAGEKGDRGPPGLDGRSGLD GKPGAAGPSGPNGAAGKAGDPGRDGLPGLRGEQGLPGPSGPPGLPGK PGEDGKPGLNGKNGEPGDPGEDGRKGEKGDSGASGREGRDGPKGER GAPGILGPQGPPGLPGPVGPPGQGFPGVPGGTGPKGDRGETGSKGEQ GLPGERGLRGEPGSVPNVDRLLETAGIKASALREIVETWDESSGSFLPV PERRRGPKGDSGEQGPPGKEGPIGFPGERGLKGDRGDPGPQGPPGLAL GERGPPGPSGLAGEPGKPGIPGLPGRAGGVGEAGRPGERGERGEKGER GEQGRDGPPGLPGTPGPPGPPGPKVSVDEPGPGLSGEQGPPGLKGAKG EPGSNGDQGPKGDRGVPGIKGDRGEPGPRGQDGNPGLPGERGMAGP EGKPGLQGPRGPPGPVGGHGDPGPPGAPGLAGPAGPQGPSGLKGEPG ETGPPGRGLTGPTGAVGLPGPPGPSGLVGPQGSPGLPGQVGETGKPGA PGRDGASGKDGDRGSPGVPGSPGLPGPVGPKGEPGPTGAPGQAVVGL PGAKGEKGAPGGLAGDLVGEPGAKGDRGLPGPRGEKGEAGRAGEPGD PGEDGQKGAPGPKGFKGDPGVGVPGSPGPPGPPGVKGDLGLPGLPGAP GVVGFPGQTGPRGEMGQPGPSGERGLAGPPGREGIPGPLGPPGPPGSV GPPGASGLKGDKGDPGVGLPGPRGERGEPGIRGEDGRPGQEGPRGLT GPPGSRGERGEKGDVGSAGLKGDKGDSAVILGPPGPRGAKGDMGERG PRGLDGDKGPRGDNGDPGDKGSKGEPGDKGSAGLPGLRGLLGPQGQP GAAGIPGDPGSPGKDGVPGIRGEKGDVGFMGPRGLKGERGVKGACGL DGEKGDKGEAGPPGRPGLAGHKGEMGEPGVPGQSGAPGKEGLIGPKG DRGFDGQPGPKGDQGEKGERGTPGIGGFPGPSGNDGSAGPPGPPGSV GPRGPEGLQGQKGERGPPGERVVGAPGVPGAPGERGEQGRPGPAGPR GEKGEAALTEDDIRGFVRQEMSQHCACQGQFIASGSRPLPSYAADTAG SQLHAVPVLRVSHAEEEERVPPEDDEYSEYSEYSVEEYQDPEAPWDSD DPCSLPLDEGSCTAYTLRWYHRAVTGSTEACHPFVYGGCGGNANRFG TREACERRCPPRVVQSQGTGTAQD |
| 6 | Nucleic acid coding rCOL7A1 | atgacgctgcggcttctggtggccgcgctctgcgccgggatcctggcagaggcgccccga gtgcgagcccagcacagggagagagtgacctgcacgcgcctttacgccgctgacattgtg ttcttactggatggctcctcatccattggccgcagcaatttccgcgaggtccgcagctttctcg aagggctggtgctgcctttctctggagcagccagtgcacagggtgtgcgctttgccacagt gcagtacagcgatgacccacggacagagttcggcctggatgcacttggctctgggggtga tgtgatccgcgccatccgtgagcttagctacaaggggggcaacactcgcacaggggctgc aattctccatgtggctgaccatgtcttcctgccccagctggcccgacctggtgtccccaaggt ctgcatcctgatcacagacgggaagtcccaggacctggtggacacagctgcccaaaggct gaaggggcagggggtcaagctatttgctgtggggatcaagaatgctgaccctgaggagc tgaagcgagttgcctcacagcccaccagtgacttcttcttcgtcaatgacttcagcatctt gaggacactactgcccctcgtttcccggagagtgtgcacgactgctggtggcgtgcctgtg acccgacctccggatgactcgacctctgctccacgagacctggtgctgtctgagccaagca |
| | | gccaatccttgagagtacagtggacagcggccagtggccctgtgactggctacaaggtcc agtacactcctctgacggggctgggacagccactgccgagtgagcggcaggaggtgaac gtcccagctggtgagaccagtgtgcggctgcggggtctccggccactgaccgagtaccaa gtgactgtgattgccctctacgccaacagcatcggggaggctgtgagcgggacagctcgg accactgccctagaagggccggaactgaccatccagaataccacagcccacagcctcctg gtggcctggcggagtgtgccaggtgccactggctaccgtgtgacatggcgggtcctcagt ggtgggcccacacagcagcaggagctgggccctgggcagggttcagtgttgctgcgtga cttggagcctggcacggactatgaggtgaccgtgagcaccctatttggccgcagtgtggg gcccgccacttccctgatggctcgcactgacgcttctgttgagcagaccctgcgcccggtca tcctgggccccacatccatcctcctttcctggaacttggtgcctgaggcccgtggctaccggt tggaatggcggcgtgagactggcttggagccaccgcagaaggtggtactgccctctgatg tgacccgctaccagttggatgggctgcagccgggcactgagtaccgcctcacactctacac tctgctggagggccacgaggtggccacccctgcaaccgtggttcccactggaccagagct gcctgtgagccctgtaacagacctgcaagccaccgagctgcccgggcagcgggtgcgag tgtcctggagcccagtccctggtgccacccagtaccgcatcattgtgcgcagcacccaggg ggttgagcggaccctggtgcttcctgggagtcagacagcattcgacttggatgacgttcag gctgggcttagctacactgtgcgggtgtctgctcgagtgggtccccgtgagggcagtgcca gtgtcctcactgtccgccgggagccggaaactccacttgctgttccagggctgcgggttgtg gtgtcagatgcaacgcgagtgagggtggcctggggacccgtccctggagccagtggattt cggattagctggagcacaggcagtggtccggagtccagccagacactgcccccagactct actgccacagacatcacagggctgcagcctggaaccacctaccaggtggctgtgtcggta ctgcgaggcagagaggagggccctgctgcagtcatcgtggctcgaacggacccactggg cccagtgaggacggtccatgtgactcaggccagcagctcatctgtcaccattacctggacc agggttcctggcgccacaggatacagggtttcctggcactcagcccacggcccagagaaa tcccagttggtttctggggaggccacggtggctgagctggatggactggagccagatact gagtatacggtgcatgtgagggcccatgtggctggcgtggatggcccccctgcctctgtg gttgtgaggactgcccctgagcctgtgggtcgtgtgtcgaggctgcagatcctcaatgcttc cagcgacgttctacggatcacctgggtaggggtcactggagccacagcttacagactggc ctggggccggagtgaaggcggccccatgaggcaccagatactcccaggaaacacagac tctgcagagatccggggtctcgaaggtggagtcagctactcagtgcgagtgactgcacttg tcggggaccgcgagggcacacctgtctccattgttgtcactacgccgcctgaggctccgcc agccctggggacgcttcacgtggtgcagcgcggggagcactcgctgaggctgcgctggg agccggtgcccagagcgcagggcttccttctgcactggcaacctgagggtggccaggaa cagtcccgggtcctggggcccgagctcagcagctatcacctggacgggctggagccagc gacacagtaccgcgtgaggctgagtgtcctagggccagctggagaagggccctctgcag aggtgactgcgcgcactgagtcacctcgtgttccaagcattgaactacgtgtggtggacac ctcgatcgactcggtgactttggcctggactccagtgtccagggcatccagctacatcctat cctggcggccactcagaggccctggccaggaagtgcctgggtccccgcagacacttccag ggatctcaagctcccagcgggtgacagggctagagcctggcgtctcttacatcttctccctg acgcctgtcctggatggtgtgcggggtcctgaggcatctgtcacacagacgccagtgtgcc cccgtggcctggcggatgtggtgttcctaccacatgccactcaagacaatgctcaccgtgc ggaggctacgaggagggtcctggagcgtctggtgttggcacttgggcctcttgggccaca ggcagttcaggttggcctgctgtcttacagtcatcggccctccccactgttcccactgaatgg ctcccatgaccttggcattatcttgcaaaggatccgtgacatgccctacatggacccaagtg ggaacaacctgggcacagccgtggtcacagctcacagatacatgttggcaccagatgctc ctgggcgccgccagcacgtaccaggggtgatggttctgctagtggatgaacccttgagag gtgacatattcagccccatccgtgaggcccaggcttctgggcttaatgtggtgatgttggga |
| | | atggctggagcggacccagagcagctgcgtcgcttggcgccgggtatggactctgtccag accttcttcgccgtggatgatgggccaagcctggaccaggcagtcagtggtctggccaca gccctgtgtcaggcatccttcactactcagccccggccagagccctgcccagtgtattgtcc aaagggccagaagggggaacctggagagatgggcctgagaggacaagttgggcctcc tggcgaccctggcctcccgggcaggaccggtgctcccggcccccaggggccccctggaa gtgccactgccaagggcgagaggggcttccctggagcagatgggcgtccaggcagccct ggccgcgccgggaatcctgggacccctggagcccctggcctaaagggctctccagggttg cctggccctcgtggggacccgggagagcgaggacctcgaggcccaaagggggagccgg gggctcccggacaagtcatcggaggtgaaggacctgggcttcctgggcggaaagggg accctggaccatcgggccccctggacctcgtggaccactgggggacccaggaccccgt ggccccccagggcttcctggaacagccatgaagggtgacaaaggcgatcgtggggagc ggggtccccctggaccaggtgaaggtggcattgctcctggggagcctgggctgccgggtc ttcccggaagccctggaccccaaggccccgttggcccccctggaaagaaaggagaaaaa ggtgactctgaggatggagctccaggcctcccaggacaacctgggtctccgggtgagca gggcccacggggacctcctggagctattggccccaaaggtgaccggggctttccagggc ccctgggtgaggctggagagaagggcgaacgtggacccccaggcccagcgggatcccg ggggctgccaggggttgctggacgtcctggagccaagggtcctgaagggccaccagga cccactggccgccaaggagagaagggggagcctggtcgccctggggaccctgcagtgg tgggacctgctgttgctggacccaaaggagaaaagggagatgtggggcccgctgggccc agaggagctaccggagtccaaggggaacggggcccacccggcttggttcttcctggaga ccctggccccaagggagaccctggagaccggggtcccattggccttactggcagagcag gacccccaggtgactcagggcctcctggagagaagggagaccctgggcggcctggccc cccaggacctgttggcccccgaggacgagatggtgaagttggagagaaaggtgacgag ggtcctccgggtgacccgggtttgcctggaaaagcaggcgagcgtggccttcggggggc acctggagttcgggggcctgtgggtgaaaagggagaccagggagatcctggagaggat ggacgaaatggcagccctggatcatctggacccaagggtgaccgtggggagccgggtcc cccaggacccccgggacggctggtagacacaggacctggagccagagagaagggaga gcctggggaccgcggacaagagggtcctcgagggcccaagggtgatcctggcctccctg gagcccctggggaaaggggcattgaagggtttcggggacccccaggcccacaggggga cccaggtgtccgaggcccagcaggagaaaagggtgaccggggtccccctgggctggat ggccggagcggactggatgggaaaccaggagccgctgggccctctgggccgaatggtg ctgcaggcaaagctggggacccagggagagacgggcttccaggcctccgtggagaaca gggcctccctggcccctctggtccccctggattaccgggaaagccaggcgaggatggcaa acctggcctgaatggaaaaaacggagaacctggggaccctggagaagacgggaggaa gggagagaaaggagattcaggcgcctctgggagagaaggtcgtgatggccccaagggt gagcgtggagctcctggtatccttggaccccaggggcctccaggcctcccagggccagtg ggccctcctggccagggttttcctggtgtcccaggaggcacgggccccaagggtgaccgt ggggagactggatccaaaggggagcagggcctccctggagagcgtggcctgcgagga gagcctggaagtgtgccgaatgtggatcggttgctggaaactgctggcatcaaggcatct gccctgcgggagatcgtggagacctgggatgagagctctggtagcttcctgcctgtgccc gaacggcgtcgaggccccaagggggactcaggcgaacaggcccccaggcaaggag ggccccatcggctttcctggagaacgcgggctgaagggcgaccgtggagaccctggccc tcaggggccacctggtctggcccttggggagaggggcccccccgggccttccggccttgc cggggagcctggaaagcctggtattcccgggctcccaggcagggctgggggtgtggga gaggcaggaaggccaggagagaggggagaacggggagagaaaggagaacgtgga gaacagggcagagatggccctcctggactccctggaacccctgggccccccggaccccct ggccccaaggtgtctgtggatgagccaggtcctggactctctggagaacagggaccccct |
| | | ggactcaagggtgctaagggggagccgggcagcaatggtgaccaaggtcccaaagga gacaggggtgtgccaggcatcaaaggagaccggggagagcctggaccgaggggtcag gacggcaacccgggtctaccaggagagcgtggtatggctgggcctgaagggaagccgg gtctgcagggtccaagaggcccccctggcccagtgggtggtcatggagaccctggacca cctggtgccccgggtcttgctggccctgcaggaccccaaggaccttctggcctgaaggg ggagcctggagagacaggacctccaggacggggcctgactggacctactggagctgtgg gacttcctggaccccccggcccttcaggccttgtgggtccacaggggtctccaggtttgcct ggacaagtgggggagacagggaagccgggagccccaggtcgagatggtgccagtgga aaagatggagacagagggagccctggtgtgccagggtcaccaggtctgcctggccctgt cggacctaaaggagaacctggccccacgggggcccctggacaggctgtggtcgggctcc ctggagcaaagggagagaagggagcccctggaggccttgctggagacctggtgggtga gccgggagccaaaggtgaccgaggactgccagggccgcgaggcgagaagggtgaag ctggccgtgcaggggagcccggagaccctggggaagatggtcagaaaggggctccag gacccaaaggtttcaagggtgacccaggagtcggggtcccgggctcccctgggcctcctg gccctccaggtgtgaagggagatctgggcctccctggcctgcccggtgctcctggtgttgtt gggttcccgggtcagacaggccctcgaggagagatgggtcagccaggccctagtggag agcggggtctggcaggccccccagggagagaaggaatcccaggacccctggggccacc tggaccaccggggtcagtgggaccacctggggcctctggactcaaaggagacaaggga gaccctggagtagggctgcctgggccccgaggcgagcgtggggagccaggcatccggg gtgaagatggccgccccggccaggagggaccccgaggactcacgggcccctggcag caggggagagcgtggggagaagggtgatgttgggagtgcaggactaaagggtgacaa gggagactcagctgtgatcctggggcctccaggcccacggggtgccaagggggacatg ggtgaacgagggcctcggggcttggatggtgacaaaggacctcggggagacaatgggg accctggtgacaagggcagcaagggagagcctggtgacaagggctcagccgggttgcc aggactgcgtggactcctgggaccccagggtcaacctggtgcagcagggatccctggtg acccgggatccccaggaaaggatggagtgcctggtatccgaggagaaaaaggagatgt tggcttcatgggtccccggggcctcaagggtgaacggggagtgaagggagcctgtggcc ttgatggagagaagggagacaagggagaagctggtcccccaggccgccccgggctggc aggacacaaaggagagatgggggagcctggtgtgccgggccagtcgggggcccctgg caaggagggcctgatcggtcccaagggtgaccgaggctttgacgggcagccaggcccc aagggtgaccagggcgagaaaggggagcggggaaccccaggaattgggggcttccca ggccccagtggaaatgatggctctgctggtcccccagggccacctggcagtgttggtccca gaggccccgaaggacttcagggccagaagggtgagcgaggtccccccggagagagag tggtgggggctcctggggtccctggagctcctggcgagagaggggagcaggggcggcc agggcctgccggtcctcgaggcgagaagggagaagctgcactgacggaggatgacatc cggggctttgtgcgccaagagatgagtcagcactgtgcctgccagggccagttcatcgcat ctggatcacgacccctccctagttatgctgcagacactgccggctcccagctccatgctgtg cctgtgctccgcgtctctcatgcagaggaggaagagcgggtaccccctgaggatgatgag tactctgaatactccgagtattctgtggaggagtaccaggaccctgaagctccttgggata gtgatgacccctgttccctgccactggatgagggctcctgcactgcctacaccctgcgctgg taccatcgggctgtgacaggcagcacagaggcctgtcacccttttgtctatggtggctgtgg agggaatgccaaccgttttgggacccgtgaggcctgcgagcgccgctgcccaccccgggt ggtccagagccaggggacaggtactgcccaggactga |

## Claims

1. Extracellular vesicles (EVs) or composition thereof for use as a medicament, wherein said EVs are produced by a genetically modified cell comprising one or more recombinant nucleic acid sequence(s) expressing recombinant Paired Box 6 (rPAX6) and/or recombinant type VII Collagen (rCOL7A1), and wherein said EVs contain (i) rPAX6 proteins and/or rPAX6 mRNAs; and/or (ii) rCOL7A1 proteins and/or rCOL7A1 mRNAs.

2. Extracellular vesicles (EVs) or composition thereof for use in the treatment or the prevention of an ocular surface blinding disorder (OSBD), wherein said EVs are produced by a genetically modified cell comprising one or more recombinant nucleic acid sequence(s) expressing recombinant Paired Box 6 (rPAX6) and/or recombinant type VII Collagen (rCOL7A1); and wherein said EVs contain (i) rPAX6 proteins and/or rPAX6 mRNAs; and/or (ii) rCOL7A1 proteins and/or rCOL7A1 mRNAs.

3. EVs or composition thereof for use according to claim 2, wherein the OSBD is a condition associated with corneal ulceration, a condition associated with conjunctivalization of the cornea and/or a condition associated with fibrosis of the cornea.

4. EVs or composition thereof for use according to claim 3, wherein the condition is a limbal stem cell deficiency (LSCD).

5. EVs or composition thereof for use according to claim 4, wherein the LSCD is the consequence of:
- a lesion of the cornea, such as burn of the cornea, physical lesions of the cornea;
- a chronic inflammation of the cornea, such as severe dry eye, mucous membrane pemphigoid, toxic epidermal necrolysis; or
- a congenital diseases of the cornea, such as congenital aniridia, aniridia-associated keratopathy (AAK) or recessive dystrophic epidermolysis bullosa (RDEB).

6. EVs or composition thereof for use according to claim 3, wherein the condition is neurotrophic keratitis, recurrent corneal erosion syndrome or mild dystrophic epidermolysis bullosa.

7. EVs or composition thereof for use according to any of the preceding claims, wherein the EVs prevent opacification of the cornea and/or improve corneal healing.

8. EVs or composition thereof for use according to any of the preceding claims, wherein the EVs are produced by an immortalized cell line.

9. EVs or composition thereof for use according to any of the preceding claims, wherein the cell is an induced pluripotent stem cell (iPS cell) or a mesenchymal stromal cell (MSC), such as a bone marrow-derived MSC (BDMSC) or an umbilical cord MSC (UCMSC).

10. EVs or composition thereof for use according to any of the preceding claims, wherein the cell is a UCMSC.

11. EVs or composition thereof for use according to any of the preceding claims, wherein the EVs or composition thereof is administered in the eye.
